# EUROPÄISCHE PATENTSCHRIFT

(11) **EP 0 939 641 B1**
(45) Veröffentlichungstag und Bekanntmachung des Hinweises auf die Patenterteilung: **28.05.2003**
(21) Anmeldenummer: 97944899.0
(22) Anmeldetag: 26.09.1997
(51) Int. Cl.: A61K 33/40, A61K 7/48, A61K 9/16, A61K 47/48

(54) **TOPISCHE MITTEL ZUR PROPHYLAXE ODER BEHANDLUNG BAKTERIELLER HAUTINFEKTIONEN**
TOPICAL PRODUCTS AS PROPHYLACTIC OR CURATIVE AGENTS FOR BACTERIAL SKIN INFECTIONS
PRODUITS TOPIQUES COMME AGENTS PROPHYLACTIQUES OU CURATIFS POUR LES INFECTIONS CUTANEES BACTERIENNES

(30) Priorität: 30.09.1996 DE 19640364
(43) Veröffentlichungstag der Anmeldung: 08.09.1999
(73) Patentinhaber: BASF AKTIENGESELLSCHAFT, 67056 Ludwigshafen (DE)
(72) Erfinder: BREITENBACH, Jörg, D-68199 Mannheim (DE); FUSSNEGGER, Bernhard, D-67489 Kirrweiler (DE); LANG, Siegfried, D-67071 Ludwigshafen (DE)
(74) Vertreter: Kinzebach, Werner, Dr.
(86) Internationale Anmeldenummer: EP9705291
(87) Internationale Veröffentlichungsnummer: WO98014199

(56) Entgegenhaltungen:
- EP-A- 0 117 612
- EP-A- 0 246 380
- WO-A-91/07184
- WO-A-94/15648

## Beschreibung

Die vorliegende Erfindung betrifft topische Mittel zur Prophylaxe und Behandlung bakterieller Hautinfektionen, die wenigstens einen Polymerkomplex enthalten, der im wesentlichen aus Wasserstoffperoxid, einem zur Komplexbildung mit Wasserstoffperoxid geeigneten Polymer, gegebenenfalls einer weiteren bakteriziden Verbindung und gegebenenfalls einem Metallsalz oder -Kolloid aufgebaut ist.

Die bakterizide Wirkung des Wasserstoffperoxids wird in. vielfältiger Weise für die Desinfektion von Flüssigkeiten und Gegenständen sowie für die topische Therapie infektiöser Erkrankungen im human- und im veterinärmedizinischen Bereich genutzt. Seine topische Anwendung kann sowohl in Form flüssiger Zubereitungen, beispielsweise wäßriger Lösungen zum Spülen von Körperhöhlen oder Fisteln, in Form fester Zubereitungen, z.B. als Komplexe des Wasserstoffperoxids mit Harnstoff, oder in Form von Salzen oder Verbindungen, die bei Hydrolyse oder Zerfall Wasserstoffperoxid bilden, erfolgen. Die bakterizide Wirkung solcher Zubereitungen erstreckt sich auf grampositive und gramnegative Kokken, Bazillen oder Spirochäten sowie auf eine Reihe von Hefen und Pilzen. Darüber hinaus wird Wasserstoffperoxid aufgrund seiner reinigenden und hämostatischen Wirkung auch für virale Infekte und Entzündungen, die nicht von Mikroorganismen hervorgerufen werden, angewendet.

Die prinzipielle Einschränkung bei der Anwendung von Wasserstoffperoxid ist jedoch seine Neigung zur Zersetzung bei Einwirkung von Wärme oder Licht oder in Gegenwart von Verunreinigungen wie Staub, verschiedenen Metallsalzen sowie alkalisch reagierenden Substanzen. Zwar beruht seine bakterizide Wirkung auf diesen Zersetzungsreaktionen, die Lager- und Gebrauchsfähigkeit seiner Zubereitungen wird jedoch eingeschränkt, da der Gehalt an Wasserstoffperoxid abnimmt.

Es hat sich gezeigt, daß Wasserstoffperoxid in Form von Komplexen mit Polymeren, vorzugsweise Polyvinylpyrrolidonen, stabilisiert werden kann. Solche Komplexe sind bekannt und beispielsweise in US-A 3,376,110, US-A 3,480,557, US-A 5,077,047, US-A 5,108,742, WO-A 91/07184 und WO-A 92/17158 beschrieben. Diese Komplexe sind in der Regel feste stabile Pulver, die sich leicht handhaben lassen und in eine Vielzahl von Zubereitungen eingearbeitet werden können. Ihre Verwendung für die Behandlung der Akne vulgaris ist in der US-A 5,130,124 beschrieben. Als nachteilig kann sich bei diesen Komplexen auswirken, daß die Freisetzung des Wasserstoffperoxids von der Bindungsstärke an das Polymer abhängt, die Ent-faltung seiner Wirkung jedoch von seinem Zerfall und damit von den im System enthaltenen Zersetzungskatalysatoren (Staubpartikel, basische Verunreinigungen, Metallspuren) abhängt.

Für eine umfassende Therapie bakterieller Hautinfektionen ist es oftmals erforderlich, die bakterizide Wirkung des Wasserstoffperoxids mit weiteren therapeutischen Wirkungen zu kombinieren. So werden bei der kosmetischen Aknebehandlung oftmals keratolytisch wirkende Substanzen wie Salicylsäure oder Schwefel (siehe G.A. Novak in "Die kosmetischen Präparate", Band 1, Verlag für chemische Industrie, Augsburg, 1982, S. 202 ff) oder Vitamin A-Säure eingesetzt. Ferner ist bekannt, daß α-Hydorxycarbonsäuren keratolytisch wirken (siehe W. Smith, SÖFW-Journal, 121. Jahrgang 14/95, S. 1013 ff). Die genannten Substanzen können jedoch zu Hautreizungen, Hautrötungen oder Allergien, seltener auch zu starken Entzündungsreaktionen führen. Andererseits ist bekannt, daß α-Hydroxysäuren oder Salicylsäure Komplexe mit Polyvinylpyrrolidon bilden (siehe D. Horn und W. Ditter, J. Pharm. Sci. 71, 1982, 1021 ff).

Es ist weiterhin bekannt, daß Polyvinylpyrrolidon als Schutzpolymer für metallische Kolloid-Lösungen, beispielweise von Kupfer, Silber (Hirai et al., Makromol. Chem. Rapid Commun. 5 (1984) 381), Palladium, Gold, Rhodium oder Platin, eingesetzt werden kann. Esumi et al. beschreiben die Herstellung kolloidaler Silberlösungen in Anwesenheit von Vinylalkohol und N-Vinylpyrrolidon (J. Appl. Polym. Sci. 44 (1992) 1003) oder Polyvinylpyrrolidon-Homopolymeren (Hirai et al. J. Macromol. Sci. Chem. A13 (1979) 633). Auch bimetallische Kolloide, insbesondere zum Einsatz als Katalysatoren, wurden von Wang et al. (Polymer Bulletin 25 (1991) 139) beschrieben.

Es ist ferner bekannt, daß Silberionen in Form von Silbersalzen toxikologisch unbedenkliche Antiseptika mit breitem Wirkspektrum darstellen. So wird 1 %-ige Silbernitratlösung Neugeborenen zur Credé-Prophylaxe (Vermeidung von Gonoblennorrhoe) direkt nach der Geburt in den Bindehautsack appliziert.

Die EP-A 0 246 380 offenbart einen Polyvinylpyrrolidon-Silbersulfadiazin-Hydrogelwundverband. Das Gel wird mittels Elektronenstrahl erzeugt; es enthält zur Farbstabilisierung Magnesiumtrisilikat und wahlweise Wasserstoffperoxid.

Die WO 94/15648 beschreibt ein Mittel zur Reinigung von Kontaktlinsen, das festes Polyvinylpyrrolidon/Wasserstoffperoxid und einen festen Neutralisator, wie bestimmte Metallkatalysatoren, enthält.

Die EP-A 0 117 612 offenbart ein Mundspülmittel, das eine wässrige Lösung von Wasserstoffperoxid und eines Polyvinylpyrrolidon-Iod-Komplexes enthält.

Die WO 93/00884 beschreibt ein Mundhygienemittel, das eine mit einem Silberkolloid stabilisierte Wasserstoffperoxidlösung enthält.

Der vorliegenden Erfindung liegt die Aufgabe zugrunde, ein topisches Mittel zur Behandlung bakterieller oder viraler Hautinfektionen bereitzustellen, das einerseits eine kontrollierte Wirkung des bakteriziden Bestandteils gewährleistet und gleichzeitig weitere therapeutisch sinnvolle Wirkungen zeigt, ohne daß die bekannten Nachteile des Standes der Technik auftreten.

Diese Aufgabe konnte überraschenderweise gelöst werden durch polymergebundenes Wasserstoffperoxid, wobei im Polymer zusätzlich ein Metallkolloid oder Metallsalz gebunden ist.

Die vorliegende Erfindung betrifft daher ein Mittel zur Prophylaxe oder Behandlung bakterieller oder viraler Hautinfektionen in Form eines Pulvers, eines Konzentrats, einer Salbe, Creme, eines Gels oder Sprays oder eines Lippenstiftes, enthaltend wenigstens einen Polymerkomplex, der, bezogen auf das Gesamtgewicht des Polymerkomplexes, im wesentlichen aufgebaut ist aus
a) 5 bis 30 Gew.-% Wasserstoffperoxid
b) 50 bis 94 Gew.-% wenigstens eines zur Bindung des Wasserstoffperoxid geeigneten Polymers,
c) 0 bis 15 Gew.-% gegebenenfalls einer weiteren therapeutisch aktiven Verbindung und
d) 0,005 bis 5 Gew.-% eines Metallkolloids oder Metallsalzes eines Metalls, das ausgewählt ist unter Cu, Ag, Au, Rh, Ir, Pd, Pt,
wobei der Polymerkomplex erhältlich ist, indem man
i) eine Lösung des Polymers mit einer Lösung von Wasserstoffperoxid und einer Lösung des Metallsalzes oder einer Dispersion des Metallkolloids und gegebenenfalls einer Lösung der Verbindung c) sprühtrocknet oder sprühgranuliert, oder
ii) für den Fall, daß es sich bei dem Polymer um eine unlösliche Form eines N-Vinyllactam-Polymerisats handelt, das Polymer im Wirbelbett mit Wasserstoffperoxid, dem Metallsalz oder Metallkolloid und gegebenenfalls der Verbindung c) umsetzt.

Unter C₁-Cₙ-Alkyl versteht man im folgenden lineare, verzweigte oder cyclische Alkylgruppen mit 1 bis n Kohlenstoffatomen. Beispiele sind Methyl, Ethyl, n-Propyl, i-Propyl, n-Butyl, 2-Butyl, i-Butyl, t-Butyl, n-Hexyl, 2-Hexyl, 2-Ethylhexyl oder n-Decyl, Cyclopentyl oder Cyclohexyl. Unter C₁-Cₙ-Alkylen versteht man lineare oder verzweigte Alkyleneinheiten, beispielsweise Methylen, Ethylen, Ethyliden, 1,1-, 1,2-, 1,3-, 2,2-Propylen, 1,1-, 1,2-, 1,3-, 1,4-, 2,2-, 2,3-Butylen. Arylgruppen sind Phenyl oder Naphthyl, die gegebenenfalls 1 bis 3 C₁-C₄-Alkylgruppen oder Halogenatome als Substituenten tragen.

Bei dem polymeren Bestandteil b) der im erfindungsgemäßen Mittel enthaltenen Polymerkomplexe handelt es sich vorzugsweise um ein Homo- oder Copolymer eines oder mehrerer N-Vinyllactame. Bevorzugte N-Vinyllactame sind N-Vinylpyrrolidon, N-Vinylpiperidon, N-Vinylcaprolactam, N-Vinylmorpholin-3-on, N-Vinyloxazolidin-4-on und deren Mischungen. Als Comonomere kommen insbesondere N-Vinylheterocyclen in Frage, z.B. Vinylpyridine oder Vinylimidazole, die gegebenenfalls einen oder mehrere C₁-C₄-Alkylreste oder Phenylreste tragen. Als Beispiele seien genannt: N-Vinylimidazol sowie 2-Methyl-1-vinylimidazol, 4-Methyl-1-vinylimidazol, 5-Methyl-1-vinylimidazol, 2-Ethyl-1-vinylimidazol, 2-Propyl-1-vinylimidazol, 2-Isopropyl-1-vinylimidazol, 2-Phenyl-1-vinylimidazol, 2-vinylpyridin, 4-Vinylpyridin sowie 2-Methyl-5-vinylpyridin.

Weiterhin können eingesetzt werden C₁-C₈-Alkylvinylether, z.B. Methylvinylether, Ethylvinylether, n-Propylvinylether, i-Propylvinylether, n-Butylvinylether, i-Butylvinylether, t-Butylvinylether, 2-Ethylhexylvinylether, Vinylester von C₁-C₁₀-Alkyl- oder C₆-C₁₀-Arylcarbonsäuren, z.B. Vinylacetat, Vinylpropionat, Vinylbutyrat, Vinylhexanoat, Vinyl-2-ethylhexanoat, Vinyldecanoat, Vinyllaurat, Vinylstearat oder Vinylbenzoat. Weiterhin kommen Ester der Acrylsäure oder der Methacrylsäure mit C₁-C₁₂-Alkanolen, vorzugsweise C₁-C₄-Alkanolen in Frage. Beispiele hierfür sind Methyl(meth)acrylat, Ethyl(meth)acrylat, n-Butyl(meth)acrylat. Weitere mögliche Comonomere sind konjugierte C₄-C₈-Diene wie Butadien oder Isopren, Vinylaromaten wie Styrol, α-Methylstyrol oder Vinyltoluole und kationisch modifizierte Vinylmonomere. Bei letzteren handelt es sich beispielsweise um monoethylenisch ungesättigte C₃-C₅-Carbonsäureester mit Aminoalkoholen der Formel 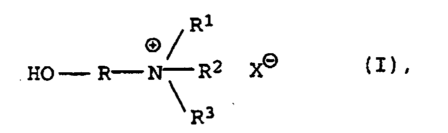 in der R für C₂-C₅-Alkylen steht, R¹, R², R³ unabhängig voneinander für H, CH₃, C₂H₅, C₃H₇ stehen und X^{⊖} ein Anion bedeutet. Geeignet sind außerdem Amide dieser Carbonsäuren, die sich von Aminen der Formel 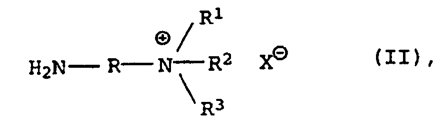 ableiten. Die Substituenten in Formel II und X^{⊖} haben die gleiche Bedeutung wie in Formel I. Geeignete Carbonsäuren sind beispielsweise Acrylsäure, Methacrylsäure, Crotonsäure, Maleinsäure(anhydrid), Fumarsäure und Itaconsäure. Als kationisch modifizierte Vinylmonomere eignen sich auch Salze oder Quaternisierungsprodukte von N-Vinylimidazol und 1-Vinyl-2-methylimidazol.

Die erfindungsgemäß verwendeten Polymerisate enthalten die genannten N-Vinyllactame in Mengen oberhalb 20 Gew.-%, vorzugsweise 30 bis 99 Gew.-% und insbesondere 35 bis 80 Gew.-% und die Comonomere in Mengen von bis zu 80 Gew.-%, vorzugsweise 1 bis 70 Gew.-% und insbesondere 20 bis 65 Gew.-% einpolymerisiert, wobei die Gewichtsteile jeweils auf das Polymerisat bezogen sind.

Weiterhin können diese Polymerisate in Mengen von bis zu 20 Gew.-%, vorzugsweise bis zu 5 Gew.-% und insbesondere 0,1 bis 3 Gew.-%, jeweils bezogen auf das Polymerisat, vernetzend wirkende Monomere mit einpolymerisiert enthalten. Geeignete Vernetzer sind in der Regel Verbindungen, die wenigstens 2, nicht konjugierte, ethylenisch ungesättigte Doppelbindungen im Molekül enthalten. Beispiele hierfür sind N,N'-Methylenbisacrylamid, Polyethylenglykoldiacrylate und Polyethylenglykoldimethacrylate, Trimethylolpropantriacrylat, Trimethylolpropantrimethacrylat, Ethylenglykoldiacrylat, Propylenglykoldiacrylat, Butandioldiacrylat. Hexandioldiacrylat, Hexandioldimethacrylat, Diacrylate und Dimethacrylate von Blockcopolymerisaten aus Ethylenoxid und Propylenoxid, zweifach bzw. dreifach mit Acrylsäure oder Methacrylsäure veresterte mehrwertige Alkohole wie Glycerin oder Pentaerythrit, Triallylamin, Tetraallylethylendiamin, Trimethylolpropandiallylether, Pentaerythrittriallylether, N,N'-Divinylethylenharnstoff und/oder N,N'-Divinylpropylenharnstoff. Weiterhin kommen als vernetzende Monomere Divinylaromaten in Frage, wie beispielsweise Divinylbenzol, aber auch Dicyclopentadien, Vinyl-(meth)acrylat, Vinylnorbornen, Tricyclodecenyl(meth)acrylat.

In einer bevorzugten Ausführungsform werden wasserlösliche Polymerisate eingesetzt. Hierbei bedeutet "wasserlöslich", daß die erfindungsgemäß verwendeten Polymerisate bei 20°C eine Löslichkeit von wenigstens 0,5 g, vorzugsweise wenigstens 2 g und insbesondere wenigstens 5 g in 100 g Wasser aufweisen. Bevorzugte Comonomere sind in diesem Fall Vinylacetat, Vinylpropionat, Methylacrylat, Ethylacrylat, n-Propylacrylat, n-Butylacrylat, Methylmethacrylat, Ethylmethacrylat, Acrylnitril, Methacrylnitril und Vinylimidazol. Ebenfalls geeignet sind Copolymerisate der N-Vinyllactame untereinander. Besonders bevorzugt werden Copolymere aus N-Vinylpyrrolidon und N-Vinylcaprolactam, N-Vinylpyrrolidon und Vinylacetat sowie Homopolymere des N-Vinylpyrrolidons. Diese wasserlöslichen Homo- und Copolymerisate weisen in der Regel K-Werte nach Fikentscher (siehe Cellulose-Chemie 13, S. 48-64 und S. 71-94 (1932)) im Bereich von 10 bis 110, vorzugsweise 20 bis 100 auf.

Die Herstellung der wasserlöslichen Polymerisate auf N-Vinyllactam-Basis ist an sich bekannt und beispielsweise in DE-A 22 18 935 oder der älteren Anmeldung P 196 09 864.5 beschrieben. Ihre Herstellung erfolgt vorzugsweise durch radikalische Lösungspolymerisation in einem wäßrigen oder alkoholischen Lösungsmittel, beispielsweise in Wasser, Methanol, Ethanol, i-Propanol oder deren Mischungen.

Als Initiatoren für die radikalische Polymerisation kommen insbesondere jene in Frage, die für die radikalische Polymerisation in wäßriger Lösung geeignet sind. Besonders geeignet sind aliphatische oder cycloaliphatische Azoverbindungen, z.B. 2,2'-Azobis (isobutyronitril), 2,2'-Azobis(2-methylbutyronitril), 2,2'-Azobis(2,4-dimethylvaleronitril), 1,1'-Azobis(1-cyclohexancarbonitril), (2-Carbamoylazo)isobutyronitril, 4,4'-Azobis-(4-cyanovaleriansäure) und deren Alkalimetall- und Ammoniumsalze, Dimethyl-2,2'-azobisisobutyrat, 2,2'-Azobis[2-(2-imidazolin-2-yl)propan], 2,2'-Azobis(2-amidinopropan) und die Säureadditionssalze der beiden letztgenannten Verbindungen. Weiterhin kommen als Initiatoren Wasserstoffperoxid, Hydroperoxide in Kombination mit geeigneten Reduktionsmitteln oder Persalze in Frage. Geeignete Hydroperoxide sind beispielsweise t-Butylhydroperoxid, t-Amylhydroperoxid, Cumolhydroperoxid und Pinanhydroperoxid, jeweils in Kombination mit beispielsweise einem Salz der Hydroxymethansulfinsäure, einem Eisen(II)-Salz oder Ascorbinsäure. Geeignete Persalze sind insbesondere Alkalimetallperoxodisulfate. Die verwendete Initiatormenge, bezogen auf die Monomere, liegt im Bereich von 0,02 bis 15 mol-%, vorzugsweise 0,05 bis 3 mol-%.

Die Polymerisation wird üblicherweise bei einem neutralen pH-Wert im Bereich von 5 bis 9 durchgeführt. Sofern notwendig, wird der pH-Wert durch Zugabe einer Base wie Ammoniak oder einer Säure wie HCl oder eines Puffersystems eingestellt bzw. aufrechterhalten. Sind niedrige Molekulargewichte erwünscht, kann die Reaktion auch in Gegenwart einer das Molekulargewicht der Polymerisate regelnden Verbindung durchgeführt werden. Hierbei handelt es sich beispielsweise um Aldehyde wie Formaldehyd, Acetaldehyd, Propionaldehyd oder Allylverbindungen wie Allylalkohol. Weiterhin können Regler eingesetzt werden, die Schwefel in organisch gebundener Form enthalten. Hierbei handelt es sich beispielsweise um Butylmercaptan, n-Hexylmercaptan, n-Dodecylmercaptan, wasserlösliche Verbindungen wie beispielsweise Hydrogensulfite, Disulfite, Ethylthioglykolat, Cystein, 2-Mercaptoethanol, Mercaptoessigsäure, 3-Mercaptopropionsäure, Thioglycerin, Diethanolsulfid, Thiodiglykol, Thioharnstoff oder Dimethylsulfoxid.

Die entstandenen Polymerisat-Lösungen weisen im allgemeinen Feststoffgehalte im Bereich von 3 bis 70 Gew.-%, bevorzugt 30 bis 60 Gew.-% auf. Sie können so wie sie bei der Polymerisation anfallen ohne weitere Isolierung oder Behandlung für die erfindungsgemäße Verfahren eingesetzt oder aber durch Fällung oder Entfernung des Lösungsmittels als Trockensubstanz isoliert werden.

In einer anderen bevorzugten Ausführungsform werden unlösliche Polymerisate eingesetzt. Solche Polymerisate werden erhalten, wenn man die Monomere in Gegenwart eines der obengenannten Vernetzer polymerisiert. Die Polymerisate können jedoch auch nachträglich durch physikalische Einflüsse, wie z.B. Strahlung oder durch chemische Reaktion mit einer bi- oder polyfunktionellen Verbindung, die mit den in den Polymerisaten enthaltenen funktionellen Gruppen reagieren kann, vernetzt und damit unlöslich gemacht werden. Solche Verfahren sind dem Fachmann bekannt und in der Literatur beschrieben. Eine besonders bevorzugte Ausführungsform der unlöslichen Polymerisate sind die sogenannten Popcorn-Polymerisate (Römpp, Chemie-Lexikon, 9. Aufl. "Popcorn Polymerisate" und dort zitierte Literatur). Die Herstellung solcher Popcorn-Polymerisate ist beispielsweise in EP-A 88 964 und EP-A 438 713 beschrieben. Sie werden in der Regel durch Substanz-, Lösungs- oder Fällungspolymerisation der Monomere, vorzugsweise in Gegenwart geringer Mengen eines Vernetzers (0,1-4 Gew.-%, bezogen auf Monomere) hergestellt.

Als weitere therapeutisch wirksame Bestandteile c) enthalten die erfindungsgemäß verwendeten Polymerkomplexe gegebenenfalls Verbindungen, die ausgewählt sind unter Aldehyden, vorzugsweise Dialdehyden, α-Hydroxycarbonsäuren, Arylcarbonsäuren, Aryldicarbonsäuren, Hydroxyarylcarbonsäuren oder hydroxysubstituierten Aromaten. Bevorzugt werden solche Substanzen, die bei topischer Anwendung pharmazeutisch verträglich sind. Bevorzugte Aldehyde sind z.B. Glutaraldehyd oder Glyoxal. Unter den α-Hydroxycarbonsäuren finden vorzugsweise Glykolsäure, Milchsäure, Hydroxyoctansäure, Äpfelsäure, Brenztraubensäure und Zitronensäure Verwendung. Geeignete aromatische Carbonsäuren sind beispielsweise Benzoesäure, phthalsäure, Isophthalsäure oder Terephthalsäure. Geeignete Hydroxyarylcarbonsäuren sind beispielsweise Salicylsäure, 3-Hydroxybenzoesäure, 4-Hydroxybenzoesäure, 2,4-Dihydroxybenzoesäure, 3,4-Dihydroxybenzoesäure und 3,5-Dihydroxybenzoesäure. Wie bereits oben erwähnt, werden die genannten Verbindungen vermutlich vom Polymerisat komplex gebunden.

Die erfindungsgemäß verwendeten Polymerkomplexe enthalten als Metallkomponente d) ein Metallsalz oder Metallkolloid des Kupfers, Silbers, Golds, Rhodiums, Iridiums, Palladiums oder Platins. Besonders bevorzugt sind Kolloide oder Salze des Kupfers oder des Silbers, insbesondere des Silbers. Geeignete Silbersalze sind beispielsweise Silbernitrat, Silberacetat, Silberlactat, Silberphosphat, Silberchlorid, Silberbromid, Silberhydroxid, Silbercarbonat, Silberoxid, Silberperiodat oder der Natriumchlorid-Silberchloridkomplex (Na[AgCl₂]). Silberkolloide sind beispielsweise dadurch erhältlich, daß man wäßrige Lösungen eines geeigneten Silbersalzes mit einem Reduktionsmittel wie Wasserstoff, Ascorbinsäure, Ribose, Glucose, Hydrazin, einem Aldehyd oder einem Alkohol behandelt (siehe Römpp, Chemie-Lexikon, 9. Aufl. "Kolloide").

Die Polymerkomplexe der erfindungsgemäßen Mittel enthalten Wasserstoffperoxid in Mengen von 5 bis 30 Gew.-%, vorzugsweise 5 bis 23 Gew.-% und insbesondere 6 bis 15 Gew.-%, jeweils bezogen auf den fertigen Polymerkomplex. Der Polymerisatgehalt beträgt 50 bis 94 Gew.-%, vorzugsweise 74 bis 94 Gew.-% und insbesondere 83 bis 94 Gew.-%. Der Gehalt an weiteren therapeutisch wirksamen Substanzen c) liegt im Bereich von 0 bis 15 Gew.-%, vorzugsweise 0,0005 bis 10 Gew.-% und insbesondere 0,01 bis 3 Gew.-%. Der Metallgehalt in den Polymerkomplexen beträgt 0,005 bis 5 Gew.-%, vorzugsweise 0,005 bis 3 Gew.-% und insbesondere 0,01 bis 2 Gew.-%.

Die Herstellung der Polymerkomplexe kann auf verschiedene Arten und Weisen erfolgen und hängt von der Art der verwendeten Polymere sowie der Bestandteile c) und d) ab. Bei Verwendung löslicher Polymere hat sich die Sprühtrocknung oder Sprühgranulation bewährt. Hierbei werden vorzugsweise wäßrige Lösungen der Polymere mit Lösungen von Wasserstoffperoxid mit Lösungen der Metallsalze bzw. Dispersionen der Metallkolloide (Komponente d) und gegebenenfalls Lösungen der Wirkstoff c) unter Einsatz von Mehrstoffdüsen gemeinsam sprühgetrocknet oder granuliert. Die Lösungen können auch zuvor vermischt worden sein. Auch ist es möglich, durch Metallsalze oder Kolloide stabilisierte Wasserstoffperoxid-Lösungen einzusetzen. In einer anderen Variante wird ein Metallkolloid in Gegenwart eines Polymers aus einer wäßrigen Lösung durch eines der genannten Reduktionsmittel abgeschieden. Über die Abscheidung von Metallkolloiden in Gegenwart von Vinylpyrrolidon-Polymerisaten wurde in der Literatur berichtet (siehe oben). Die erhaltene Dispersion wird dann zusammen mit einer Wasserstoffperoxid-Lösung sprühgetrocknet. Auch hier ist es möglich, die Lösungen zuvor zu vermischen. Die verwendeten Polymerlösungen können durch Auflösen des Polymers in einem geeigneten Lösungsmittel, vorzugsweise einem wäßrig alkoholischen oder wäßrigen Lösungsmittel hergestellt werden. Auch ist es möglich, die bei der Polymerisation anfallenden Lösungen direkt zu verwenden. Im erfindungsgemäßen Verfahren wird Wasserstoffperoxid in Form 30 bis 70 gew.%-iger, vorzugsweise 30 bis 60 gew.%-iger wäßriger Lösungen eingesetzt.

Die Verfahren zur Sprühtrocknung oder Sprühgranulierung sind dem Fachmann bekannt. Auch im vorliegenden Fall kann die Gewinnung der festen Polymerkomplexe in Sprühtürmen herkömmlicher Bauart erfolgen. Als Trocknungsgase werden inerte Gase wie beispielsweise Stickstoff verwendet, die im Gegenstrom oder vorzugsweise im Gleichstrom mit den Flüssigkeitstropfen durch den Trocknungsturm geleitet werden. Die Turmeingangstemperatur des Gases liegt in der Regel bei 60 bis 180°C, vorzugsweise bei 100 bis 160°C, und die Turmausgangstemperatur bei 40 bis 100°C, vorzugsweise bei 60 bis 90°C. Der Druck liegt in der Regel im Bereich von 0,6 bis 1,5 bar, vorzugsweise erfolgt die Trocknung bei Normaldruck. Der entstandene Feststoff kann in üblicher Weise von dem Gasstrom, beispielsweise durch ein Zyklon oder Filtersäcke. abgetrennt werden. Man erhält auf diese Weise ein frei fließendes Pulver mit einem Restlösemittelgehalt < 7,5 Gew.-%, bezogen auf den fertigen Polymerkomplex. Die Teilchengröße im entstandenen Pulver liegt im allgemeinen bei 10 bis 150 µm, bei Sprühgranulation können Teilchengrößen von bis zu 450 µm erreicht werden.

Eine andere Ausführungsform der exfindungsgemäßen Komplexe besteht darin, daß man die unlöslichen Formen der N-Vinyllactam-Polymerisate im Wirbelbett mit Wasserstoffperoxid, dem Metallsalz oder Metallkolloid d) und gegebenenfalls dem Wirkstoff c) umsetzt. Auch hier kann die Metallkomponente zuvor mit der Wasserstoffperoxid-Lösung vermischt werden.

In einer weiteren Ausführungsform weisen die erfindungsgemäß verwendeten Komplexe einen schalenförmigen Aufbau auf. Dieser schalenförmige Aufbau wird dadurch erreicht, daß man die obengenannten Bestandteile a), b), c) und gegebenenfalls d) in der für den gewünschten Aufbau notwendigen Kombination oder Reihenfolge in einer geeigneten Apparatur, beispielsweise einem Dragierkessel oder einem Wirbelschichtgranulator, sukzessive auf einen festen Träger aufträgt. Auch diese Verfahren sind dem Fachmann im Prinzip bekannt. Bei diesem festen Träger handelt es sich um anorganische Oxide wie Titandioxid, Aluminiumoxid, Siliciumdioxid, Silikate, Alumosilikate, organische Trägermaterialien wie Zellulose, Stärke, unlösliche Polymerisate, vorzugsweise solche, die zur Komplexbildung mit Wasserstoffperoxid geeignet sind, insbesondere solche auf Polyvinyllactambasis. Der letztgenannte Träger kann bei der Herstellung der erfindungsgemäßen Komplexe auch als Komponente b) dienen.

Die Herstellung der erfindungsgemäßen, schichtförmig aufgebauten polymerkomplexe geschieht dadurch, daß man auf den Träger in einer der obengenannten Apparaturen die Lösungen oder Suspensionen der Komponenten a) und c) und gegebenenfalls b) und/oder d) unter den Bedingungen, wie sie für die Polymerkomplexpulver beschrieben worden sind, aufsprüht. Dieser Vorgang kann gegebenenfalls wiederholt werden, bis die gewünschten Konzentrationsverhältnisse der Komponenten eingestellt sind. Die Komponenten können über Mehrstoffdüsen gleichzeitig oder aber sukzessive in beliebiger Reihenfolge aufgesprüht werden.

Soll eine pH-Wert abhängige Freisetzung des Wasserstoffperoxids, der Wirkstoffe c) und des Metalls erfolgen, können die Komplexe mit einem polymeren Filmbildner, der bei einem bestimmten pH-Wert in Lösung geht oder quillt, überzogen werden. Durch geeignete Wahl dieser Filmbildner kann eine Freisetzung sowohl im sauren wie im alkalischen Milieu initiiert werden. Solche Filmbildner können bei schalenförmig aufgebauten Komplexen auch die einzelnen Schichten gegeneinander abgrenzen, so daß z.B. eine erste Schicht bei einem neutralen pH den Wirkstoff freisetzt und eine innere Schicht erst oberhalb oder unterhalb eines bestimmten pH-Wertes. Geeignete polymere Filmbildner sind aus der Galenik bekannt. Dabei handelt es sich beispielsweise um Hydroxypropylcellulose, Hydroxypropylmethylcellulose, Vinylpyrrolidon-Vinylacetatcopolymere, Celluloseacetatphthalat, Hydroxypropylmethylcellulosephthalat, Schellack, Copolymerisate von Acrylsäure oder Methacrylsäure mit (Meth)acrylsäureestern, z.B. Copolymere aus Ethylacrylat und Methacrylsäure (z.B. Kollicoat® MAE 30D, oder Endragile®) oder Copolymerisate von Dimethylaminomethacrylsäure mit neutralen Methacrylsäureestern.

Bevorzugte Ausführungsformen der erfindungsgemäßen Komplexe sind wie folgt aufgebaut:
- Kern: Polymer b) mit Komponente a); Schale: Polymer b) mit Komponente c) und gegebenenfalls Komponente d);
- Kern: Polymer b) mit Komponente a); Schale 1: polymerer Filmbildner (s.o.); Schale 2: Polymer b) mit Komponente c) und gegebenenfalls Komponente d);
- Kern: Polymer b) mit den Komponenten a) und d); Schale 1: polymerer Filmbildner (s.o.); Schale 2: Polymer b) mit Komponente c) und gegebenenfalls d).

Bei der Herstellung der erfindungsgemäßen Polymerkomplexe, sei es als Polymerpulver oder als schalenförmiges Granulat, können weitere Bestandteile zugegen sein, die die Verarbeitung der erfindungsgemäßen Polymerkomplexe vereinfachen oder deren Wirkungsspektrum erweitern. Beispielsweise kann man bei der Herstellung der erfindungsgemäßen Komplexe Tenside zusetzen, die anschließend im Komplex verbleiben. Diese können in Kontakt mit Keimen die Wirkung des eigentlichen Desinfektions-Systems Wasserstoffperoxid/Metall/Polymer erhöhen und als Lösungsvermittler bzw. Netzmittel dienen. Geeignete Tenside können sowohl kationischer, anionischer als auch nichtionischer Natur sein. Beispiele hierfür sind Natriumdodecylsulfat, Dodecyltrimethylammoniumbromid, Dimethylalklybenzylammoniumchlorid, Polysorbatfettsäureester sowie ethoxylierte Mono-, Di- und Trialkylphenole (EO-Grad: 3 bis 50, Alkylrest:C₄-C₉), ethoxylierte Fettalkohole (EO-Grad: 3 bis 50, Alkylrest C₈-C₃₆), sowie Alkali- und Ammoniumsalze von Alkylsulfaten (Alkylrest: C₈-C₁₂), von Schwefelsäurehalbestern ethoxylierter Alkanole (EO-Grad: 4 bis 30, Alkylrest: C₁₂-C₁₈) und ethoxylierter Alkylphenole (EO-Grad: 3 bis 50, Alkylrest: C₄-C₉), von Alkylsulfonsäuren (Alkylrest: C₁₂-C₁₈) und von Alkylarylsulfonsäuren (Alkylrest: C₉-C₁₈). Bevorzugte Emulgatoren sind Natriumdodecylsulfat und Polysorbatfettsäureester.

Die beschriebenen Polymerkomplexe sind als Feststoffe bei Raumtemperatur über einen langen Zeitraum hinweg ohne Verlust an Wasserstoffperoxid haltbar. Gegenüber den meisten pharmazeutisch verwendeten Formulierungsbestandteilen verhalten sie sich inert. Somit bestehen hinsichtlich ihrer Formulierung in topischen Mitteln keine Beschränkungen. Als Formulierungsbescandteile werden entsprechend der gewünschten Zubereitungsform die üblichen pharmazeutischen oder kosmetischen Träger und Hilfsmittel verwendet.

Als Formulierungsbestandteile können beispielsweise Alkohole wie Ethanol, Propanol Isopropanol, Phenoxyethanol, Phenoxy-1- und Phenoxy-2-propanol, Polyole wie Propylenglykol, Glycerin oder Polyethylenglykole, Silicone, Ester oder Glyceride von Fettsäuren, beispielsweise Isopropylmyristat, Myricylcerotinoat, Cetylpalmitat, Glyceride der Palmitinsäure, Stearinsäure, Linolsäure, Linolensäure oder Ölsäure, Phospholipide, wie Kephaline oder Lecithine, Stärken, modifizierte Stärken oder Kohlenwasserstoffe, beispielsweise Vaseline oder Paraffine, dienen. Darüber hinaus können die Formulierungen Bestandteile enthalten, die für ihre Herstellung notwendig sind, z.B. die obengenannten Tenside.

Die erfindungsgemäßen Mittel sind zur topischen Applikation geeignet. Sie, liegen als Puder, Salben, Cremes, Gele oder Sprays für die Prophylaxe bakterieller Hautinfektionen, z.B. bei der Wundversorgung, oder die Behandlung bakterieller Hautinfekte, beispielsweise bei der Akne vulgaris, vor- Weiterhin können die erfindungsgemäßen Mittel in Form von antibakteriellen Lippenstiften formuliert werden. Da die erfindungsgemäßen Mittel in der Regel Einsatzstoffe beinhalten, die pharmazeutisch anerkannt sind, bestehen hinsichtlich ihrer Anwendung im humanmedizinischen Bereich keine Bedenken.

### BEISPIELE

### Verwendetes Ausgangsmaterial

- Stabilisiertes Wasserstoffperoxid: 50 %ige wäßrige Lösung, stabilisiert mit 0.082 Gew.-% kolloidalem Silber; Handelsprodukt der Fa. Hungerbach GmbH, Mörsdorf.
- Polyvinylpyrrolidon K30: K-Wert 30 nach H. Fikentscher, Cellulose-Chemie 13 (1932) 18, 71.; Handelsprodukt der BASF.
- Polyvinylpyrrolidon, vernetzt: Crospovidone®, Handelsprodukt der BASF AG.
- Polyvinylcaprolactam: K-Wert 30
- Copolymer aus Vinylimidazol/Vinylpyrrolidon (VI/VP = 9:1) hergestellt nach folgender Vorschrift:
   In einer Rührapparatur mit aufgesetztem Rückflußkühler wurde ein Gemisch aus 9 Teilen N-Vinylimidazol (VI), 1 Teil N-Vinylpyrrolidon (VP), 0,3 Teilen N,N'-Divinylimidazolidon, 100 Teilen Wasser und 0,1 Teilen Natronlauge (5%ig) vorgelegt und unter Zusatz von 0.1 Teilen eines vernetzten, wenig quellbaren Polymerisats auf Basis von VI und/oder VP im Stickstoffstrom auf 70°C erhitzt. Bei dieser Temperatur wurde 6 h polymerisiert. Das erhaltene Fällungspolymerisat wurde abgesaugt, mit Wasser gründlich gewaschen und bei 60°C getrocknet. Man erhielt ein weißes, körniges Produkt in einer Ausbeute von 96,5%.
- Copolymer aus Ethylacrylat/Methacrylsäure (EA/MAS: 1/1): Kollicoat MAE30 D der BASF AG.

### Analytik

Der Wasserstoffperoxidgehalt der erfindungsgemäßen Polymerkomplexe wurde durch Titration mit Kaliumpermanganat bestimmt.
Die Bestimmung des Metallgehalts der erfindungsgemäßen Polymerkomplexe erfolgte durch Atomabsorptionsspektrometrie.
Der Wassergehalt der Polymerkomplexe wurde durch Karl-Fischer Titration bestimmt.

### Homogene Polymer-Wassertstoffperoxid-Silber-Komplexe

### (Beispiele 1-3)

### Beispiel 1 (Herstellung durch Sprühtrocknung)

In einem Trockenturm (D 900 mm; H 1400 mm) wurde mit Hilfe einer Zweistoffdüse bei einem Druck von 1,5 bar eine Lösung aus

| | |
|---|---|
| 500 g | Polyvinylpyrrolidon K30 |
| 153 g | Wasserstoffperoxid (50 %-ige wäßrige Lösung stabilisiert mit 0.126 g kollidalem Silber) und |
| 1347 g | Wasser |

versprüht. Die Trocknung erfolgte mittels Stickstoff bei 1 bar und einer Turmeingangstemperatur von 160°C sowie einer Turmausgangstemperatur von 70°C. Das entstandene Pulver wurde über einen Zyklon vom Gasstrom abgetrennt. Das erhaltene Pulver wies einen Peroxidgehalt von 12.9 Gew.-%, einen Silbergehalt von 0.019 Gew.-% und einen Wassergehalt von 3 Gew.-% auf.

### Beispiel 2 (Herstellung durch Sprühtrocknung)

150 g Polyvinylpyrrolidon und 5.6 g Silbernitrat wurden in 500 ml Ethanol gelöst und 60 min zum Rückfluß erhitzt. Anschließend gab man 60 g einer 25 %igen, wäßrigen Wasserstoffperoxid-Lösung zu. Die erhaltene Lösung wurde wie in Bsp. 1 sprühgetrocknet. Das erhalten Pulver wies einen Peroxidgehalt von 8 Gew.-%, einen Silbergehalt von 1,9 Gew.-% und einen Lösemittelgehalt von 1 Gew.-% auf.

### Beispiel 3 (Herstellung durch Wirbelbettrocknung)

Die Herstellung durch Wirbelbettrocknung erfolgt in einem Granulierzylinder, der nach unten durch einen Lochboden mit Siebauflage (Maschenweite 10-500 µm) und nach oben durch 4 Filtersäcke, welche alle 15 sec durch Druckluf freigeblasen werden, abgeschlossen ist. 28 cm oberhalb des Siebbodens befindet sich eine dem Siebboden entgegengerichtete Zweistoffdüse. Die Dosierung der Wasserstoffperoxid-Silber-Lösung erfolgt mittels einer Schlauchpumpe und Zugabegeschwindigkeiten von 2,5 bis 100 g/min/1000 g Polymer. Die Einsatzmenge an Polymer beträgt 100 bis 4000 g. Der Gasdurchsatz wird durch eine Abluftklappe reguliert und beträgt 120 m³/h bis 150 m³/h. Als Pozessgas wird Stickstoff verwendet. Die Zulufttemperatur liegt im Bereich von 25 bis 80°C, die Ablufttemperatur im Bereich von 25 bis 70°C.

250 g Polyvinylcaprolactam wurden bei einem Gasstrom von 120 m³/h im Wirbelbett vorgelegt und bei 50°C mit 153 g einer 50 Gew.-%igen wäßrigen Wasserstoffperoxid-Lösung, die 0,126 g kolloidales Silber enthielt, besprüht (10 g/min). Anschließend wurde im Gasstrom bei 50°C (150 m³/h; 20 min) getrocknet. Der Peroxidgehalt des erhaltenen Pulvers betrug 23 Gew.-%, der Silbergehalt 0,035 Gew.-% und der Wassergehalt 1 Gew.-%.

### Schalenförmige Polymer-Wasserstoffperoxid-Silber-Komplexe

### (Beispiele 4 bis 9)

### Beispiel 4 (VI/VP-Wasserstoffperoxid-Silber-Komplex)

200 g unlösliches VI/VP-Polymerisat wurden in einem Wirbelschichtgranulator nach Bsp. 3 vorgelegt und bei 60°C in 4 Intervallen mit jeweils 25 ml und dann in 3 Intervallen mit jeweils 50 ml 20 %igem Wasserstoffperoxid (jew. 20 ml/min) besprüht. Zwischen den Sprühintervallen wurde jeweils 5 min. im Gasstrom getrocknet. Auf die gleiche Weise wurde auf den erhaltenen Polymer-Wasserstoffperoxidkomplex 250 ml einer wäßrigen Silberkolloidsuspension mit einem Silbergehalt von 0.16 Gew.% gesprüht. Der erhaltene Komplex wies einen Peroxidgehalt von 16,8 Gew.-% einen Silbergehalt von 0.196 Gew.-% und einen Wassergehalt von 3,7 Gew.-% auf.

### Beispiel 5

### (Polymer-Wasserstoffperoxid-Silber-Komplex mit Filmbildner)

100 g vernetztes Polyvinylpyrrolidon wurden zunächst wie in Beispiel 4 mit 200 ml einer 15 %-igen Wasserstoffperoxidlösung besprüht. Die erhaltenen Polymer-Wasserstoffperoxid-Komplexe besprühte man in 4 Portionen mit einer Lösung aus 15 g Kollicoat MAE 30 D, 2g Triethylcitrat und 0,1 g kolloidalem Silber in 100 ml Wasser und trocknete wie in Beispiel 3. Der erhaltene Komplex wies einen Peroxidgehalt von 18 Gew.-%, einen Silbergehalt von 0,06 Gew.-% und einen Wassergehalt von 2 Gew.-% auf. Silber und Wasserstoffperoxid wurden bei einem pH-Wert von 5,5 freigesetzt.

### Beispiel 6

### (Polymer-Wasserstoffperoxid-Silber-Komplex mit Filmbildner)

100 g vernetztes Polyvinylpyrrolidon wurden zunächst wie in Beispiel 4 mit 200 ml einer 15 %-igen Wasserstoffperoxidlösung besprüht. Die erhaltenen Polymer-Wasserstoffperoxid-Komplexe besprühte man in 4 Portionen mit einer Lösung aus 15 g Kollicoat MAE 30 D und 2g Triethylcitrat. Anschließend wurde mit einer Lösung aus 0,1 g kolloidalem Silber in 100 ml Wasser besprüht und wie in Beispiel 3 getrocknet.

### Beispiel 7

### (Polymer-Wasserstoffperoxid-Silberkomplex mit Filmbildner).

Es wurde wie in Beispiel 6 verfahren, jedoch wurde anstelle des Silberkolloids eine 0,1 Gew.-%ige Lösung von Silbernitrat verwendet. Der Silbergehalt im erhaltenen Komplex betrug 0,04 Gew.-%.

### Beispiel 8

### (Polymer-Wasserstoffperoxid-Milchsäure-Komplex mit Filmbildner)

100 g vernetztes Polyvinylpyrrolidon wurden in einem Granulierzy-linder (s.o.) vorgelegt und bei 60°C in vier Intervallen mit jeweils 20 ml und dann in drei Intervallen mit jeweils 40 ml einer 15 gew.-%igen wässrigen Wasserstoffperoxidlösung besprüht (20 ml/min). Zwischen den Sprühintervallen wurde jeweils 5 min im Gasstrom getrocknet (150 m³/Stunde; 60°C).

Anschliessend sprühte man in 4 Portionen eine Lösung aus 15 g Kollicoat®MAE30D in 100 ml Wasser auf und trocknete erneut 20 min. Hiernach sprühte man eine Lösung von 1 g Milchsäure in 100 ml Wasser in 4 Portionen auf und trocknete erneut.

Der erhaltene Polymerkomplex wies einen Wasserstoffperoxidgehalt von 19 Gew.-% und einen Wassergehalt von 1 Gew.-% auf.

### Beispiel 9

### (Polymer-Wasserstoffperoxid-Silber-Komplex mit Filmbildner)

Beispiel 8 wurde wiederholt, jedoch wurden 200 ml einer 15 gew.-%igen Wasserstoffperoxidlösung, die mit 0,025 Gew.-% Silber stabilisert ist, verwendet.

Der Wasserstoffperoxidgehalt des erhaltenen Produkts betrug 20. Gew.-%, der Silbergehalt 0,024 Gew.-% und der Wassergehalt 1,5 Gew.-%.

### Formulierungen der erfindungsgemäßen Polymerkomplexe

### (Beispiele 10 bis 14)

### verwendete Komponenten

- Polyacrylsäure: Carbopol® C981, der Fa. BF Goodrich Chemical
- Ethylenoxid/Propylenoxid-Blockcopolymer (EO/PO 70/30) Mₙ 9840 bis 146000; Lutrol® F 127 der BASF.
- Polyethylenglykol: Mₙ 400; Lutrol® E 400 der BASF.
- Polyethylenglykol: Mₙ 4000; Lutrol® E 4000 der BASF
- Silkonöl: Dichte ρ (25°C) 0,95 g/cm³, Viskosität (25°C) 2,5 mm²/s, Dow Corning Fluid 344 (cyclisches Tetra(dimethyl)siloxan der Dow-Corning.
- Distärkeposphat auf Basis von Maisstärke: Mais PO4 100 K (P-Gehalt: < 0,1 Gew.-% in Trockenmasse); Fa. Hauser KG

### Beispiel 10 (Formulierung als Zahncreme)

10 g des nach Beispiel 1 hergestellten Komplexes wurden in 78 g Wasser gelöst und in einem Vakuumhomogenisator mit 2 g Carbopol® und 10 g 1,2-Propylenglykol zu einem luftblasenfreien Gel verarbeitet.

### Beispiel 11 (Gel zur Hautdesinfektion)

10 g Komplex aus Beispiel 1, 75 g Wasser, 5 g 1,2-Propylenglykol und 20 g Lutrol® F127 wurden unterhalb 10°C in der für Beispiel 6 beschriebenen Weise zu einem Gel verarbeitet.

### Beispiel 12 (Formulierung als Salbe)

20 g Komplex aus Beispiel 1 wurden in einer Mischung aus 50 g Lutrol® E 400 und 5 g Wasser gelöst und auf 55-60°C erwärmt. Anschließend arbeitete man bei dieser Temperatur unter Rühren 25 g Lutrol® E 4000 ein und ließ unter Rühren abkühlen.

### Beispiel 13 (Formulierung als Konzentrat für Mundspülungen)

25 g Komplex aus Beispiel 1 wurden in einer Mischung aus 1 g 1,2-Propylenglykol, 9 g Ethanol und 65 g Wasser gelöst.

### Beispiel 14 (Formulierung als Puderspray)

2,5 g Komplex aus Beispiel 1 wurden in einem Mikronisator mikronisiert und zusammen mit 1 g Silikonöl (s.o.) und 2,5 g Maisstärke-Diphosphat in ein Druckgefäß gegeben. Anschließend befüllte man mit 5 g Pentan und 2,2 g Propan/Butan.

## Patentansprüche

1. Topisches Mittel zur Prophylaxe oder Behandlung bakterieller oder viraler Hautinfektionen in Form eines Puders, eines Konzentrats, einer Salbe, Creme, eines Gels oder Sprays, oder eines Lippenstifts, enthaltend wenigstens einen Polymerkomplex, der, bezogen auf das Gesamtgewicht des Polymerkomplexes, im Wesentlichen aufgebaut ist aus
a) 5 bis 30 Gew.-% Wasserstoffperoxid,
b) 50 bis 94 Gew.-% wenigstens eines zur Bindung des Wasserstoffperoxids geeigneten Polymers,
c) 0 bis 15 Gew.-% einer weiteren therapeutisch aktiven Verbindung, und
d) 0,005 bis 5 Gew.-% eines Metallkolloids oder Metallsalzes eines Metalls, das ausgewählt ist unter Cu, Ag, Au, Rh, Ir, Pd, Pt,
wobei der Polymerkomplex erhältlich ist, indem man
i) eine Lösung des Polymers mit einer Lösung von Wasserstoffperoxid und einer Lösung des Metallsalzes oder einer Dispersion des Metallkolloids und gegebenenfalls einer Lösung der Verbindung c) sprühtrocknet oder sprühgranuliert, oder
ii) für den Fall, daß es sich bei dem Polymer um eine unlösliche Form eines N-Vinyllactam-Polymerisats handelt, das Polymer im Wirbelbett mit Wasserstoffperoxid, dem Metallsalz oder Metallkolloid und gegebenenfalls der Verbindung c) umsetzt.

2. Topisches Mittel nach Anspruch 1, enthaltend Polymerkomplexe, in denen der Bestandteil b) ein Polymer auf Basis von N-Vinyllactamen ist.

3. Topisches Mittel nach Anspruch 2, enthaltend Polymerkomplexe, in denen der Bestandteil b) aufgebaut ist aus
20 bis 100 Gew.-% wenigstens eines N-Vinyllactams,
0 bis 80 Gew.-% wenigstens eines copolymerisierbaren, monoethylenisch ungesättigten Monomeren und
0 bis 20 Gew.-% wenigstens eines vernetzend wirkenden Monomeren.

4. Topisches Mittel nach Anspruch 2 oder 3, in denen das N-Vinyllactam ausgewählt ist unter N-Vinylpyrrolidon, N-Vinylpiperidon, N-Vinylcaprolactam, N-Vinylmorpholin-3-on, N-Vinyloxazolidin-4-on.

5. Topisches Mittel nach einem der vorhergehenden Ansprüche, in denen der polymere Bestandteil b) ein Homo- oder Copolymer des N-Vinylpyrrolidons oder des N-Vinylcaprolactams mit einem K-Wert im Bereich von 10 bis 110 ist.

6. Topisches Mittel nach einem der vorhergehenden Ansprüche, wobei die therapeutisch aktive Substanz ausgewählt ist unter Aldehyden, α-Hydroxycarbonsäuren, polyhydroxylierten Aromaten oder Hydroxylcarbonsäuren.

7. Topisches Mittel nach Anspruch 1, worin die Komponente d) ein Metallsalz oder Kolloid des Silbers ist.

## Claims

1. A topical composition for the prophylaxis or treatment of bacterial or viral infections of skin in the form of a dusting powder, a concentrate, an ointment, a cream, a gel or a spray or of a lip salve, comprising at least one polymer complex essentially composed of, based on the total weight of the polymer complex,
a) 0.5 to 30% by weight of hydrogen peroxide
b) 50 to 94% by weight of at least one polymer suitable for binding the hydrogen peroxide,
c) 0 to 15% by weight of another therapeutically active compound and
d) 0.005 to 5% by weight of a metal colloid or metal salt of a metal selected from Cu, Ag, Au, Rh, Ir, Pd, Pt,
the polymer complex being obtainable by
i) spray drying or spray granulating a solution of the polymer with a solution of hydrogen peroxide and a solution of the metal salt or a dispersion of the metal colloid and, where appropriate, a solution of compound c), or
ii) in the case where the polymer is an insoluble form of an N-vinyllactam polymer, reacting the polymer in a fluidized bed with hydrogen peroxide, the metal salt or metal colloid and, where appropriate, compound c).

2. A topical composition as claimed in claim 1, comprising polymer complexes in which component b) is a polymer based on N-vinyllactams.

3. A topical composition as claimed in claim 2, comprising polymer complexes in which component b) is composed of 20 - 100% by weight of at least one N-vinyllactam, 0 - 80% by weight of at least one copolymerizable monoethylenically unsaturated monomer and 0 - 20% by weight of at least one crosslinking monomer.

4. A topical composition as claimed in claim 2 or 3, in which the N-vinyllactam is selected from N-vinylpyrrolidone, N-vinylpiperidone, N-vinylcaprolactam,
N-vinyl-3-morpholinone, N-vinyl-4-oxazolidinone.

5. A topical composition as claimed in any of the preceding claims, in which the polymeric component b) is a homo- or copolymer of N-vinylpyrrolidone or of N-vinylcaprolactam with a K value in the range from 10 to 110.

6. A topical composition as claimed in any of the preceding claims, wherein the therapeutically active substance is selected from aldehydes, α-hydroxy carboxylic acids, polyhydroxylated aromatic compounds or hydroxyarylcarboxylic acids.

7. A topical composition as claimed in claim 1, in which component d) is a metal salt or colloid of silver.

## Revendications

1. Produit topique pour la prophylaxie ou le traitement des infections bactériennes ou virales de la peau, sous la forme d'une poudre, d'un concentré, d'une pommade, d'une crème, d'un gel ou d'un aérosol, ou d'un bâton pour les lèvres, contenant au moins un complexe polymère et qui, par rapport au poids total du complexe polymère, est constitué essentiellement de
a) 5 à 30 % en poids de peroxyde d'hydrogène,
b) 50 à 94 % en poids d'au moins un polymère capable de fixer le peroxyde d'hydrogène,
c) 0 à 15 % en poids d'un autre composé possédant une activité thérapeutique et
d) 0,005 à 5 % en poids d'un métal colloïdal ou d'un sel métallique d'un métal choisi parmi Cu, Ag, Au, Rh, Ir, Pd, Pt,
le complexe polymère étant obtenu
i) par séchage par atomisation ou granulation par pulvérisation d'une solution du polymère avec une solution de peroxyde d'hydrogène et une solution du sel métallique ou une dispersion du métal colloïdal et le cas échéant une solution du composé c),
ou bien
ii) dans le cas ou le polymère consiste en une forme insoluble d'un polymère d'un N-vinyllactame, par réaction du polymère dans un lit tourbillonnaire avec le peroxyde d'hydrogène, le sel métallique ou le métal colloïdal et le cas échéant le composé c).

2. Produit topique selon la revendication 1, contenant des complexes polymères dans lesquels le constituant b) est un polymère à base de N-vinyllactame.

3. Produit topique selon la revendication 2, contenant des complexes polymères dans lesquels le constituant b) est formé à partir de :
20 à 100 % en poids d'au moins un N-vinyllactame,
0 à 80 % en poids d'au moins un monomère à insaturation monoéthylénique copolymérisable et
0 à 20 % en poids d'au moins un monomère réticulant.

4. Produit topique selon la revendication 2 ou 3, dans lequel le N-vinyllactame est choisi parmi la N-vinylpyrrolidone, la N-vinylpipéridone, le N-vinylcaprolactame, la N-vinylmorpholin-3-one, la N-vinyloxazolidin-4-one.

5. Produit topique selon l'une des revendications qui précèdent dans lequel le constituant polymère b) est un homo- ou co-polymère de la N-vinylpyrrolidone ou du N-vinylcaprolactame ayant un indice K dans l'intervalle de 10 à 110.

6. Produit topique selon l'une des revendications qui précèdent dans lequel la substance possédant l'activité thérapeutique est chois parmi des aldéhydes, des acides alpha-hydroxycarboxyliques, des composés aromatiques polyhydroxylés ou des acides hydroxycarboxyliques.

7. Produit topique selon la revendication 1, dans lequel le composant b) est un sel métallique de l'argent ou de l'argent colloïdal.
